Europäisches Patentamt

European Patent Office

Office européen des brevets

(11) Veröffentlichungsnummer: **0 294 714 B1**

# (12) EUROPÄISCHE PATENTSCHRIFT

(45) Veröffentlichungstag der Patentschrift: **22.07.92**

(21) Anmeldenummer: **88108870.2**

(22) Anmeldetag: **03.06.88**

(51) Int. Cl.⁵: **G01N 33/96**

(54) **Verwendung von Polyvinylpyrrolidon (PVP) zur Trübungsminderung von Seren, Seren enthaltend PVP und Verfahren zu deren Herstellung.**

(30) Priorität: **09.06.87 DE 3719196**

(43) Veröffentlichungstag der Anmeldung:
**14.12.88 Patentblatt 88/50**

(45) Bekanntmachung des Hinweises auf die
Patenterteilung:
**22.07.92 Patentblatt 92/30**

(84) Benannte Vertragsstaaten:
**AT BE CH DE FR GB IT LI LU NL SE**

(56) Entgegenhaltungen:
**EP-A- 0 014 252**
**EP-A- 0 141 922**
**US-A- 3 764 478**

**ENCYCLOPEDIA OF POLYMER SCIENCE AND
ENGINEERING, Band 7, 1987, John Wiley &
Sons, New York, NY (US); Seite 277**

**CHEMICAL ABSTRACTS, Band 99, Nr.17, 24
Oktober 1983, Columbus, OH (US); Seite 461,
Nr.138179r**

(73) Patentinhaber: **BEHRINGWERKE Aktiengesellschaft
Postfach 1140
W-3550 Marburg 1(DE)**

(72) Erfinder: **Steuer, Dagmar
Am Sonnenhang 9
W-3550 Marburg(DE)**

(74) Vertreter: **Tergau, Ulrich, Dr. et al
HOECHST AKTIENGESELLSCHAFT Zentrale
Patentabteilung Postfach 80 03 20
W-6230 Frankturt am Main 80(DE)**

EP 0 294 714 B1

**Beschreibung**

Die Erfindung betrifft Kontrollseren auf humaner oder tierischer Basis in getrockneter und rekonstituierbarer Form, wobei dem flüssigen Serum vor der Trocknung Polyvinylpyrrolidon (PVP) zugesetzt wird und dadurch eine Verringerung der Trübung im rekonstituierten Serum erreicht wird, Verfahren zu ihrer Herstellung sowie ihre Verwendung zur Qualitätskontrolle in der klinisch-chemischen Diagnostik.

Unter Kontrollseren sind Seren humanen oder tierischen Ursprungs mit gegebenenfalls veränderter, jedoch serumähnlicher Zusammensetzung zu verstehen, die Serumbestandteile, beispielsweise Proteine, Enzyme, enzymatisch bestimmbare Substrate und Elektrolyte in bekannter Konzentration enthalten und zur Kontrolle von Bestimmungsmethoden für diese Serumbestandteile geeignet sind. Als Kontrollseren im Sinne der Erfindung sind auch Standardseren zu verstehen.

Verfahren zur Herstellung derartiger Kontrollseren einschließlich der Einstellung einzelner Bestandteile auf gewünschte Gehalte sind bekannt.

Um die Haltbarkeit labiler Komponenten wie beispielsweise Enzymen oder Lipoproteinen zu gewährleisten, können Kontrollseren lyophilisiert und bei niedriger Temperatur gelagert werden. Unerwünschte Nebeneffekte einer Lyophilisation sind Trübungen, die nach Rekonstitution der Kontrollseren durch Veränderung des Lösungsverhaltens vor allem der Lipoproteine auftreten. Diese Trübungen stören häufig bei spektrophotometrischen Methoden, so daß ein Probenleerwert zusätzlich erforderlich ist. Besondere Probleme bereitet die Trübung bei Messungen im Bereich von 340 nm, in dem vor allem solche Enzymaktivitätsbestimmungen durchgeführt werden, die auf einer NADH/NAD-Messung beruhen. Die an sich schon hohe Extinktion von NADH wird durch die Trübung noch erhöht, sodaß häufig in einem Bereich gemessen werden muß, in dem präzise Messungen nicht möglich sind. Die Ergebnisse werden ungenauer und hängen stark von der Güte des Photometers ab.

Es sind bereits Verfahren zur Vermeidung von Trübungen bekannt. In DE-P 31 07 060 ist der Zusatz organischer nicht zuckerartiger Substanzen wie Methanol, Alanin, Triäthylenglycol, Valin, Acetat, Lactat oder Natrium-2-hydroxymethylbutyrat beschrieben. Ein solcher Zusatz kann im Falle von Alanin und Methylbutyrat Störungen von Enzymreaktionen zur Folge haben. Ein Methanolzusatz ist allgemein gesundheitsgefährdend. Wenn Natriumacetat verwendet wird, ist das Kontrollserum nicht mehr als Universal-Kontrollserum für Elektrolytbestimmungen einsetzbar. Ein Zusatz von Ammoniumverbindungen stört bei Harnstoff-Bestimmungen. Andere Substanzen können generelle Teststörungen verursachen.

Ein weiteres Verfahren zur Vermeidung von Trübungen ist in DE-OS 33 29 952 durch Zusatz von Prolin und Na-Desoxycholat vorbeschrieben. Dieser Zusatz hat jedoch den Nachteil, daß er zu Artefakten bei der Proteinauftrennung mittels Elektrophorese führt und die Enteiweißung mit Trichloressigsäure z.B. bei Bestimmung von Kreatinin erschwert.

Die Erfindung hat sich daher die Aufgabe gestellt, ein Universal-Kontrollserum mit verminderter Trübungsneigung nach Rekonstitution aus einer getrockneten Form herzustellen, das die beschriebenen Nachteile der bekannten Mittel nicht besitzt.

Überraschenderweise wurde gefunden, daß sich trübungsärmere, homogenere rekonstituierte Kontrollsera herstellen lassen, wenn dem Kontrollserum vor der Trocknung Polyvinylpyrrolidon (PVP) zugesetzt wird. Dadurch wird das Serum insgesamt nicht nur homogener sondern auch die Präzision von Konzentrations- und Aktivitätsbestimmungen der im Kontrollserum enthaltenen Parameter verbessert, d. h. die Wiederfindung der deklarierten Sollwerte wird für den Anwender von Qualitätskontrollsera erleichtert.

Gegenstand der Erfindung ist daher ein Kontrollserum auf humaner oder tierischer Basis in getrockneter und rekonstituierbarer Form, wobei dem flüssigen Serum vor der Trocknung Polyvinylpyrrolidon (PVP) zugesetzt wird.

Weiterhin wurde gefunden, daß eine Trübung des Kontrollserums, dem Lipide wie Cholesterin und Triglyceride zugesetzt werden, ebenfalls mit Polyvinylpyrrolidon als weiterer Zusatz vermindert und die Homogenität des rekonstituierten Serums verbessert wird.

Gegenstand der Erfindung ist auch ein Verfahren zur Herstellung eines Kontrollserums wie oben beschrieben, wobei dem flüssigen Serum vor der Trocknung PVP zugegeben wird.

Ferner ist Gegenstand der Erfindung die Verwendung eines Kontrollserums wie oben beschrieben zur Qualitätskontrolle klinisch interessanter Parameter in der klinisch chemischen Diagnostik.

Die wirksame Konzentration des PVP mit einem Molekulargewicht von 10.000 - 750.000 liegt zwischen 1-20 g/l. Vorzugsweise werden 2-6 g/l, besonders bevorzugt 3 g/l mit einem Molekulargewicht 25.000 - 30.000 eingesetzt. Das erfindungsgemäße Verfahren eignet sich zur Herstellung von Klinisch-chemischen Kontrollseren, d.h. von Präparaten, die zur Qualitätskontrolle klinisch interessanter Serumparameter wie Enzym-, Substrat-, Lipid-, Metabolit-, Hormonoder Elektrolytgehalt verwendet werden sollen. Es eignet sich ebenfalls zur Herstellung eines Kontrollserums mit dosierten Lipiden (Cholesterin, Triglyceride).

2

Die Extinktion des mit destilliertem Wasser rekonstituierten, getrockneten Kontrollserums mit dem erfindungsgemäßen Zusatz wurde mit einem Linienspektralphotometer bei 546 nm in einer Küvette mit 1 cm Schichtdicke gemessen und verglichen mit der Extinktion desselben Kontrollserums ohne Zusatz.

Unter Rekonstitution versteht man die Lösung eines Trokkengutes in der Menge Lösungsmittel, in der es vor der Trocknung enthalten war.

Das folgende Beispiel erläutert die Erfindung.

## BEISPIEL

Als Basis für Kontrollserum wurde gepooltes Humanserum von gesunden Spendern und Rinderserum verwendet. Zu einem Teil der Seren wurde Eigelb-Extrakt und Rindercholesterinkonzentrat zugegeben, wobei Konzentration an Triglyceriden und Cholesterin erhalten wurden, die höher waren als die in der folgenden Tabelle unter den Spalten "Zugabe von Lipiden" angegebenen Werten. Alle derart erhaltenen Proben von Seren wurden wiederum in zwei Anteile geteilt, wobei der erste Anteil ohne Zusatz belassen wurde, dem zweiten Anteil 3 g/l Polyvinylpyrrolidon, Molekulargewicht 25.000-30.000 der Firma Merck AG, Darmstadt, Art. Nr. 7443, in fester Form zugesetzt und darin gelöst wurde.

Alle Proben wurden anschließend keimfrei filtriert, in Flaschen abgefüllt und lyophilisiert. Nach Rekonstitution mit destilliertem Wasser wurden Cholresterin, Triglyceride und die Trübung gemessen.

Die Tabelle gibt die Ergebnisse der Messungen von Cholesterin, Triglyceriden und als Maß der Trübung die Extinktion und die Variationsbreite der Extinktion von Flasche zu Flasche (Homogenität) bei 546 nm wieder. Ihr ist zu entnehmen, daß der Zusatz von Polyvinylpyrrolidon bei allen Proben eine deutliche Verringerung der Trübung und des Variationskoeffizienten (VK), ein Maß für die genannte Variationsbreite bewirkt.

| S E R E N | Cholesterin mmol/1 | Triglyceride mmol/1 | ohne Polyvinylpyrrolidon Ext. 546 nm (n = 10) | | mit Polyvinylpyrrolidon Ext. 546 nm (n = 10) | |
|---|---|---|---|---|---|---|
| | | | $\bar{x}$ | vk % | $\bar{x}$ | vk % |
| **Human-Serumbasis** | | | | | | |
| ohne Zugabe | 3,18 | 0,68 | 0,440 | 13,0 | 0,148 | 2,0 |
| | 3,64 | 0,86 | 0,461 | 13,8 | 0,250 | 1,5 |
| Zugabe von Substraten, Enzymen und Elektrolyten | 2,84 | 0,72 | 0,674 | 4,4 | 0,303 | 2,3 |
| | 3,20 | 1,03 | 0,801 | 13,9 | 0,435 | 2,5 |
| Zugabe von Lipiden | 3,23 | 2,03 | 0,628 | 17,2 | 0,227 | 3,7 |
| | 3,49 | 2,58 | 0,692 | 5,0 | 0,298 | 3,2 |
| | 5,79 | 0,73 | 0,636 | 8,2 | 0,249 | 2,8 |
| | 5,76 | 2,49 | 0,789 | 6,0 | 0,401 | 4,9 |
| **Rinderserum** | | | | | | |
| Zugabe von Lipiden | 1,37 | 0,18 | 0,242 | 10,8 | 0,220 | 5,8 |
| | 5,43 | 2,20 | 0,983 | 16,3 | 0,667 | 4,7 |

**Patentansprüche**

1. Kontrollserum auf humaner oder tierischer Basis in getrockneter und rekonstituierbarer Form, dadurch gekennzeichnet, daß dem flüssigen Serum vor der Trocknung Polyvinylpyrrolidon (PVP) zugesetzt wird.

2. Verfahren zur Herstellung eines Kontrollserums gemäß Anspruch 1 dadurch gekennzeichnet, daß dem flüssigen Serum vor der Trocknung PVP zugegeben wird.

3. Verwendung eines Kontrollserums gemäß Anspruch 1 zur Qualitätskontrolle klinisch interessanter Parameter in der klinisch chemischen Diagnostik.

4. Kontrollserum gemäß Anspruch 1 dadurch gekennzeichnet, daß das zugesetzte PVP ein Molekulargewicht von 10.000 - 750.000 hat.

5. Kontrollserum gemäß Anspruch 1 dadurch gekennzeichnet, daß dem flüssigen Serum vor der Trocknung 1-20 g/l PVP zugesetzt wird.

6. Kontrollserum gemäß Anspruch 1 dadurch gekennzeichnet, daß dem flüssigen Serum zur Aufstockung Lipide wie Cholesterin und Triglyceride zugesetzt werden.

**Claims**

1. A human- or animal-based control serum in dried and reconstitutable form, wherein polyvinylpyrrolidone (PVP) is added to the liquid serum before drying.

2. A process for the preparation of a control serum as claimed in claim 1, which comprises adding PVP to the liquid serum before drying.

3. The use of a control serum as claimed in claim 1 for the quality control of parameters of clinical interest in clinicochemical diagnosis.

4. A control serum as claimed in claim 1, wherein the added PVP has a molecular weight of 10,000-750,000.

5. A control serum as claimed in claim 1, wherein 1-20 g/l PVP is added to the liquid serum before drying.

6. A control serum as claimed in claim 1, wherein lipids such as cholesterol and triglycerides are added to the liquid serum to raise the level of such analytes.

**Revendications**

1. Sérum témoin d'origine humaine ou animale, sous forme déshydratée et reconstituable, caractérisé en ce que de la polyvinylpyrrolidone (PVP) a été ajoutée au sérum liquide, préalablement à la déshydratation.

2. Procédé pour préparer un sérum témoin selon la revendication 1, caractérisé en ce qu'on ajoute de la PVP au sérum liquide, préalablement à la déshydratation.

3. Utilisation d'un sérum témoin selon la revendication 1 pour le contrôle de la qualité de paramètres intéressants du point de vue clinique, lors du diagnostic chimique de type clinique.

4. Sérum témoin selon la revendication 1, caractérisé en ce que la PVP ajoutée a une masse moléculaire allant de 10000 à 750000.

5. Sérum témoin selon la revendication 1, caractérisé en ce qu'on ajoute 1 à 20 g/l de PVP au sérum liquide, préalablement à la déshydratation.

6. Sérum témoin selon la revendication 1, caractérisé en ce que des lipides, comme du cholestérol et des triglycérides, sont ajoutés au sérum liquide pour en augmenter le titre.